# EUROPEAN PATENT SPECIFICATION

(11) **EP 0 667 133 B1**
(45) Date of publication and mention of the grant of the patent: **07.03.2001**
(21) Application number: 93830500.0
(22) Date of filing: 14.12.1993
(51) Int. Cl.: A61F 2/24

(54) **A percutaneous implantable valve for the use in blood vessels**
Perkutan implantierbares Ventil für Blutgefässe
Valve d'implant percutané pour vaisseaux sanguins

(43) Date of publication of application: 16.08.1995
(73) Proprietor: Camilli, Sante, I-00139 Roma (IT)
(72) Inventor: Camilli, Sante, I-00139 Roma (IT)
(74) Representative: Gristina, Giorgio

(56) References cited:
- EP-A- 0 030 826
- EP-A- 0 520 126
- WO-A-91/17720
- WO-A-93/01768
- DE-A- 4 101 935
- US-A- 4 056 854

## Description

### Field of the Invention

The present invention relates to the valves for the use in living bodies.

More particularly, it relates to the artificial valves for the use in a blood vessel, internal to the blood vessel itself, in contact with the blood stream passing therethrough.

Still more particularly, though not exclusively, the present invention relates to the valves of the said type, which are intended to operate inside a vein, as well as endowed with a monocusp valving element.

As used herein, by "valving element", or "valving means", the means is meant, mounted on the support or the valve, whereby the control of the blood flow is achieved.

### Background of the Invention

There isn't any artificial valve for the use in blood vessels that is implantable at a distance at the present day.

Moreover, there isn't any artificial valve for the use in veins commercially available at the present day, notwithstanding some specific attempts, such as those disclosed by WO-A-88 00459 (LANE), WO-A-89 02254 (TAHERI) and Quijano *et al.* (WO 90/14804).

Another valve is disclosed by Andersen (International Publication Number WO 91/17720). It shows a valve prosthesis for implantation in the body which comprises a stent made from an expandable cylinder shaped thread structure comprising spaced apices. The valve is elastically collapsible and is mounted on the stent as the commissural points of the valve are secured to the projecting apices. The valve prosthesis can be compressed around balloon means of a balloon catheter and be inserted into a channel, for instance the aorta.

The main problem with such valves is the ease of thrombosis, which immobilizes the valve and, therefore, prevents it from operating.

Lane's valve isn't, strictly speaking, a valve, but only an outer prosthesis whereinto a vein segment is intussuscepted to form a venous valve, in which the blood doesn't come in contact with the prosthesis itself. It is not commercially available, it is not implantable at a distance.

Taheri's valve has an annular support which has a tendency to go out of alignement when implanted in a vein during low endoluminal pressure stages and when the vein is compressed from the outside. Its valving means are two leaflets, which aren't protected by the annular support during their motion of opening and closing from the collapse of the venous wall during low endoluminal pressure stages and when the vein is compressed from the outside. It isn't commercially available, it isn't implantable at a distance.

Quijano *et al.*'s valve is a biological valve, that is to say it is entirely made from animal tissues, and is endowed with a two-leaflet valving means. It isn't commercially available, it isn't implantable at a distance.

Various types of artificial valves made for being used in heart (heart valves) are known in the operative art: these valves are not applicable in veins. In fact, they are intrinsically different from venous valves for the structure or the size or the materials or the characteristics of mechanical strength or the forces necessary for their operation or the way of insertion or the method for fixing them in the desired site. In heart, and in the vessels connected therewith, there are high pressures and high instantaneous flows; moreover, the vessels have large diametres and are protected against compression from the outside by virtue of being positioned inside the thorax. On the contrary, in veins there are low pressures and low flows; moreover, the diametres of the veins that have valvular incompetence are small and these veins are easily compressible from outside under various conditions.

In order to provide an artificial valve that could operate in a vein, the same Applicant hereof suggested, in the Italian Patent Application RM91A000458 of 25^{th} June, 1991, (corresponding European Patent Application EP-A-520 126) a valve configured to allow unidirectional flow, biocompatible with veins, comprising
a hollow elongated support having a predetermined length with a periphery and a predetermined width, said length being greater than said width, said support having means for fixing itself to a vein, and
a plate carried by and within the support and movable relative to the support between a first position to allow flow of blood in one direction and a second position in which to prevent flow of blood in an opposite direction through said support,
said plate being disposed entirely within said support in both of said first and second positions.

Such a valve has only one valving element, as this is made up of the said plate, which is a monocusp; so thrombogenesis is substantially reduced and the valve is able to operate reliably.

The fact that the valving element is always inside the said support during its motion ensures that it is always protected against the collapse of the vein during low pressure stages.

### Objects, Features and Advantages of the Invention

The main object of the present invention is to provide a valve for the use in blood vessels, which is implantable at a distance.

Another object of the present invention is to provide a valve for the use in blood vessels, which is substantially less thrombogenic than the known ones.

Such objects are achieved, according to the teaching of the present invention, by utilizing a suitably bent flexible wire having elasticity and plasticity as the support of the valving means.

As used herein, by "having elasticity and plasticity" it is meant that said wire is plastically deformed when twisted into turns by an operator, but reacts elastically to the forces applied to it by the wall of a blood vessel, particularly a vein, once implanted in the blood vessel itself.

The advantages offered by the present invention are mainly that it provides a valve for the use in blood vessels which is implantable at a distance, which was never made before. This advantage is achieved by virtue of the cage structure of the wire support, which can be so made, as to be folded in such a way as to free its three-dimensional space occupation. After introducing at a distance, percutaneously, the valve into the desired site, the valve itself can be restored to its original three-dimensional configuration.

Moreover, the present invention affords a valve which is substantially less thrombogenic than the known ones: indeed, its support doesn't develop bidimensionally, still by virtue of its cage structure; then it offers a very restricted extension for the formation of thrombi.

### Subject of the Invention

Therefore, the present invention relates to a valve as specified in claim 1.

It is provided that said sail valving element is endowed with a check line fixed to said central turn. in order to prevent the valving element itself from stopping in a don't care condition.

It is also provided that said sail valving element is endowed with a control shaft incorporated in itself, again to prevent the valving element from stopping in a don't care position.

It is also provided that the valve of the present invention is endowed with hooking means for its fixation to a blood vessel wall.

A catheter to be used with the valve is endowed at its distal end with a capsule for transporting, means for grasping and loading into said capsule as well as for releasing in a desired site a valve according to the present invention.

It is provided that such a catheter comprises an inflatable balloon to obtain the occlusion of a blood vessel.

### Detailed Disclosure of the Preferred Embodiment

The present invention will be best understood on the basis of the following disclosure of its preferred embodiment, given only as a matter of example and not of restriction, with reference to the annexed drawings, wherein:
- figure 1 is a side view of an artificial valve according to the present invention, in its closure position;
- figure 2 is a front view of the same;
- figure 3 is a side view of the valve, in its opening position;
- figure 4 is a perspective view from the top of the valve;
- figures 5a to 5c show a catheter for implanting the above valve into a vein.

The support or valve body is made up of a wire mesh 1, in a plastic material or in a biocompatible metallic alloy, such as the nickel (Ni) and cobalt (Co) alloy Phynox^{®}, eventually treated with antithrombogenic processes, having a circular cross section, with a diametre of about 0.3 millimetres, flexible, elastic and plastic, shaped and twisted so as to form three turns, whereof two end ones 6, 6', substantially circular and lying on the periphery of the bases of a right cylinder, and a central one 5, with an ellipse-like contour substantially lying on a plane at about 130° relative to the said circle-like turns 6, 6'.

The so shaped valve body 1 gives the valve a cylinder cage configuration.

It is provided that the base of such a cylinder has a diametre of 6 to 14 millimetres and a height of 15 to 25 millimetres, the sizes being variable in connection with the veins whereinto the valve is to be positioned.

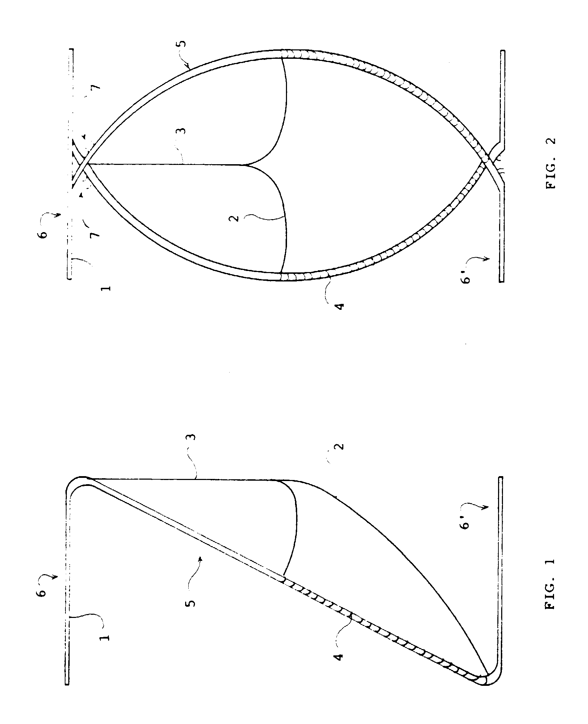

Once a suitably sized valve has been inserted into a vein, the ellipse-like turn, or main turn, 5 goes to assume an oblique position relative to the direction of the blood stream, indicated with 11 in figures 1 to 4, while the other two, circle-like turns 6, 6' will assume a position substantially perpendicular to it.

The function of the main, ellipse-like turn 5 is to constitute the support for anchoring a mobile plate or valving element 2.

The function of the two end turns 6, 6' is to keep the main turn 5 in the desired position and to impede a permanent collapse of the vein segment that contains the valve during low endoluminal pressure stages or after extrinsic compression, causing the redistension of the venous segment by means of the elastic reaction of the material which all the valve body is made up of.

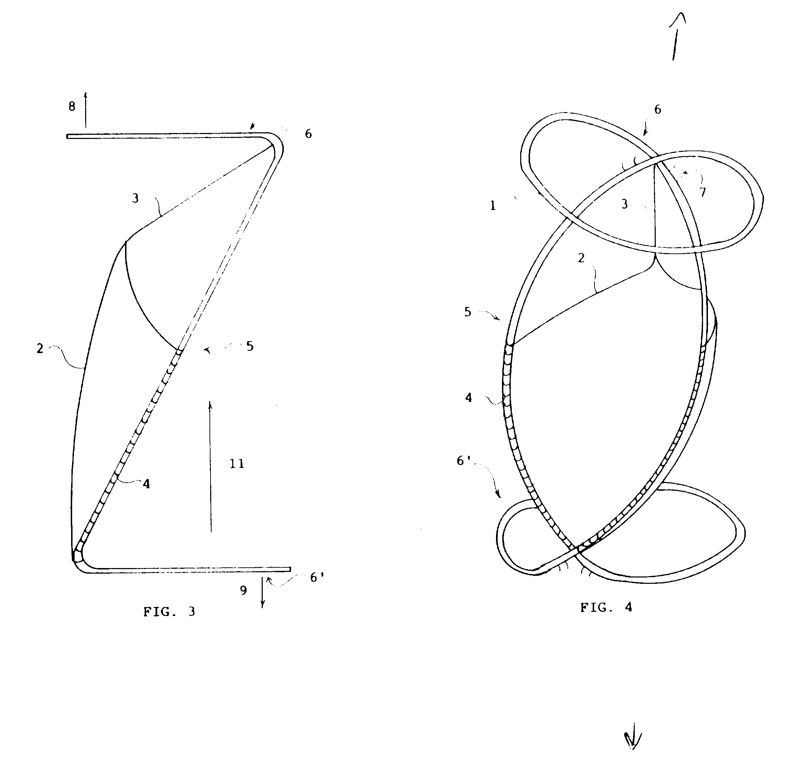

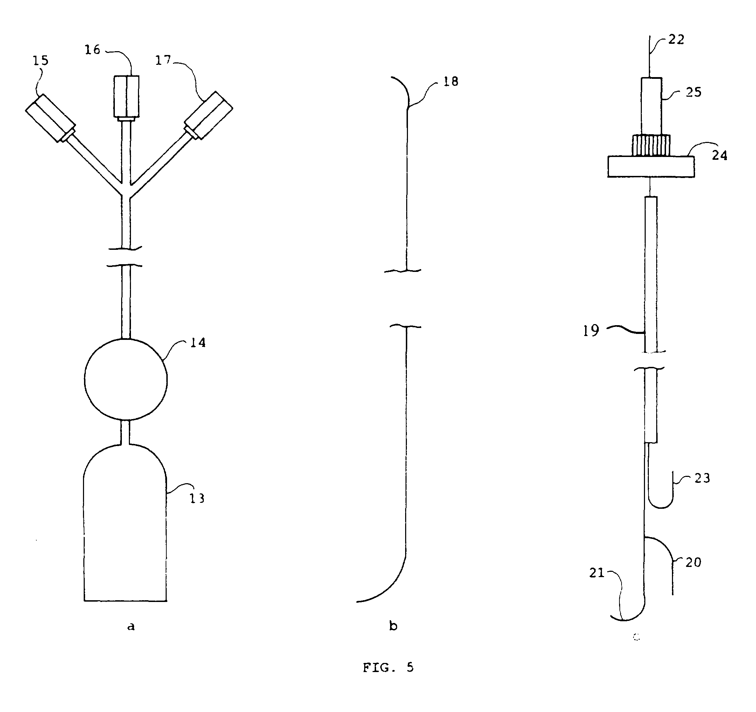

The so configured support or valve body 1 is fit for being easily deformed, by applying a traction upon the two end turns 6, 6', so obtaining the movement indicated with the arrows 8 and 9 in figure 3 that causes a temporary and incomplete linearization, or decrease of its space occupation and increase of occupied length, of the valve. Under these conditions, the valve can be loaded into the carrying capsule 13 of the introducer catheter, to be disclosed hereinafter in connection with figures 5a to 5c.

Subsequently, during its release and anyhow after any eventual deformation for extrinsic compression, the valve is able to return to its original shape.

The support 1 is endowed with suitable hooks 7, arranged on one or more points of the three turns which it is made up of, having the aim of fixing into the wall of the vein after the release and the positioning of the valve, to prevent its accidental mobilization.

The mobile plate or sail valving element 2 is made up of a plastic material, as for instance expanded polytetrafluoroethylene or polyurethane or silicone, or of a homologous biologic material, as for instance pericardium, eventually treated with one of the processes in use with antithrombogenic aim, in thin plate, as for instance 0.1 to 0.3 millimetres. The mobile plate is comprised of a sail, see figures 1 to 4. It is connected with the ellipse-like support turn 5 by means of a biocompatible, essentially non thrombogenic suture thread 4 commonly used in vascular surgery, or of another process fit for obtaining the fixation between the two elements and can be reinforced or anchored in various ways and in one or more points of its free edge to the end of helping a correct closure position thereof and of preventing its stopping in a don't care position. In the present embodiment, the sail valving element is endowed with a check line 3 anchored to the turn 5 to this end, but it could also be endowed with an incorporated shaft (not shown).

The mobile plate 2 is freely floating within the venous lumen and is moved by the pressure gradient on the two surfaces of the same. In the stage of muscular systole, when the pressure on its distal surface is greater than that on its proximal surface, the plate lifts itself and assumes the opening position as in figure 3. With the valve in this position, the blood stream 11 is directed towards the heart. In the stage of muscular diastole, when the pressure of the blood upon the proximal surface is greater than that on the distal surface, the plate returns to the closure position 2, as in figure 1, and the backflow in distal direction is stopped. The return to the correct closure position is rendered easier by the presence of the reinforcement and anchoring element 3, which keeps the mobile plate in the most effective position for the whole operation of the valve.

Having disclosed a valve according to the teaching of the present invention, an instrument for introducing and positioning it into a vein is now disclosed.

Such an instrument is comprised of an introducer catheter, shown in figures 5a to 5c.

This latter in made up of:
- a tubular catheter 12 in a plastic material, as for instance polyethylene or silicone or other, of a suitable diametre for the use of two operative ways;
- an end capsule 13, in a metal or in a plastic material, of a suitable hardness and antiadherence, of a suitable diametre of 3,3-5 mm (10 to 15 F), hollow at its interior and ending with an opening extended to all its diametre, fitted for housing the valve according to the present invention during the transport manoeuvre from the exterior to the interior of the vein, up to the established point;
- an inflatable balloon 14, arranged upstream of the capsule 13, with a connection channel coaxial with the operative one and endowed with a Luer^{®} lock connector 15, suitable for obtaining the stopping of the venous circulation and thus useful to render the angioscopic and angiographic check easier during and after the positioning of the valve;
- a flexible guide 18, of the type in use in angiography, which can be introduced into the introducer catheter through the connector 17, suitable during the stage of introduction of said introducer catheter, and
- a manoeuvre instrument 19 to 25, which can be introduced into the catheter through the way 16, for grasping the valve during both the stage of loading into the interior of the transport capsule 13 and the stage of release into the vein: it is made up of a not very flexible guide 19 endowed at its extremity with a suitable instrument 20 for fixing the valve and with a very flexible and delicate termination 21; with a guide 22, coaxial with the preceding one, it also provided with a suitable instrument 23 for grasping the valve and slideable in the two senses relative to the guide 19 to 21 of a radial check and positioning reference 24; with a screw or sliding system, or other, fit for producing the movement 26 of attraction or pushing of the guide 22, 23 relative to the guide 19 to 21.

The introducer catheter, after attracting the valve into the interior of the capsule by exploiting its flexibility features, is introduced into the venous lumen and allows the transport of the valve by intraluminal way up to the desired point and finally its release and definitive positioning.

The positioning takes place under the inspection of the surgeon's view or under fluoroscopic monitoring or other method.

The fixing of the valve into its correct position, in the interior of the vein, can be achieved by means of direct suture points, applied from the exterior onto the venous wall and comprising in one or more points the valve body 1, or through the fixing into the venous wall of the proper hooks 7 on the body of the valve. In the latter case, the valve still can be repositioned or removed and eventually substituted through percutaneous way, with a process analogous to that of introduction at a distance.

## Claims

1. A valve for use in a blood vessel, internal to the blood vessel itself, in contact with a blood stream, which is adapted to be introduced and positioned into the blood vessel up to a desired site by a catheter, the catheter being endowed at its distal end with a capsule (13) for transporting into the blood vessel and with means (19 to 25) for grasping and loading the valve into said capsule (13) as well as for releasing it in the desired site,
the valve comprising:
a body including a bent flexible wire (1) having elasticity and plasticity so as to be resilient, self-expanding and implantable remotely at the desired site;
wherein said wire is bent into at least three turns, to confine a tubular space, said at least three turns including a central turn (5) and two end turns (6, 6'), said central turn (5) being at an angle relative to said end turns (6, 6'); and
a monocusp valving element (2) mounted on the central turn (5).

2. A valve for use in a blood vessel according to Claim 1, characterized in that the valve further includes hooking means (7) for fixing the wire to blood vessel wall.

3. A valve for use in a blood vessel according to Claim 1, characterized in that the monocusp valving element (2) is endowed with a check line (3) fixed to said central turn (5).

4. A valve for use in a blood vessel according to Claim 1, characterized in that the catheter further comprises an inflatable balloon (14) to obtain the occlusion of the blood vessel.

## Patentansprüche

1. Ventil zur Verwendung in einem Blutgefäß, das sich in dem Blutgefäß selbst in Kontakt mit dem Blutstrom befindet, das adaptiert ist, um mittels eines Katheders in das Blutgefäß bis zu einer gewünschten Stelle eingeführt und positioniert zu werden, wobei der Katheter an seinem distalen Ende mit einer Kapsel (13) zum Transport in das Blutgefäß und mit Einrichtungen (19 bis 25) zum Greifen und Einbringen von diesem in die Kapsel (13) sowie zum Aussetzen desselben an der gewünschten Stelle ausgestattet ist, wobei das Ventil
einen Körper einschließlich eines gebogenen flexiblen Drahts (1) mit Elastizität und Plastizität, damit er rückfedernd, selbst-expandierend und an der gewünschten Stelle fernimplantierbar ist, wobei der Draht in mindestens drei Biegungen gebogen ist, um einen Hohlaum zu bilden, wobei die mindestens drei Biegungen eine zentrale Biegung (5) und zwei Endbiegungen (6,6') einschließen, wobei sich die zentrale Biegung (5) in einem Winkel relativ zu den Endbiegungen (6,6') befindet, und
ein Monosegelventilelement (2) umfasst, welches auf der zentralen Biegung (5) montiert ist.

2. Ventil zur Verwendung in einem Blutgefäß nach Anspruch 1, dadurch gekennzeichnet, dass das Ventil außerdem Hakeneinrichtungen (7) zum Fixieren des Drahts an einer Blutgefäßwand einschließt.

3. Ventil zur Verwendung in einem Blutgefäß nach Anspruch 1, dadurch gekennzeichnet, dass das Monosegelventilelement (2) mit einer an der zentralen Biegung (5) befestigten Prüfleitung (3) ausgestattet ist.

4. Ventil zur Verwendung in einem Blutgefäß nach Anspruch 1, dadurch gekennzeichnet, dass der Katheter außerdem einen aufblasbaren Ballon (14) umfasst, um den Verschluss des Blutgefäßes zu erhalten.

## Revendications

1. Une canule à utiliser dans un vaisseau sanguin, qui se place à l'intérieur de ce même vaisseau sanguin et entre en contact avec le flux sanguin ; qui est conçue de façon à être introduite et placée dans le vaisseau sanguin jusqu'à l'endroit désiré à l'aide d'un cathéter, le cathéter étant doté à son extrémité distale d'une capsule (13) destinée à la transporter à l'intérieur du vaisseau sanguin, et d'éléments (19 à 25) destinés à l'accrocher et à l'installer dans ladite capsule (13) et pour la libérer à l'endroit souhaité ;
la canule comprend :
un corps comportant un fil flexible courbé (1) ayant une élasticité et une plasticité qui le rendent résistant, auto-extensible et qui permettent de l'implanter n'importe où selon l'endroit souhaité ;
où ledit fil est courbé de manière à former trois boucles au moins pour constituer un espace tubulaire limité, lesdites trois boucles incluant une boucle centrale (5) et deux extrémités (6, 6'), ladite boucle centrale (5) se trouvant à un angle par rapport auxdites extrémités (6, 6') ; et
un élément tubulaire à cuspide simple (2) monté sur la boucle centrale (5).

2. Une canule à utiliser dans un vaisseau sanguin selon la Revendication 1, caractérisée par le fait que la canule est dotée de crochets (7) destinés à fixer le fil à la paroi d'un vaisseau sanguin.

3. Une canule à utiliser dans un vaisseau sanguin selon la Revendication 1, caractérisée par le fait que l'élément tubulaire à cuspide simple (2) est doté d'une ligne de contrôle (3) fixée à la boucle centrale (5).

4. Une canule à utiliser dans un vaisseau sanguin selon la Revendication 1, caractérisée par le fait que le cathéter comporte en outre un ballon gonflable (14) destiné à provoquer l'occlusion du vaisseau sanguin.
